# EUROPÄISCHE PATENTANMELDUNG

(11) **EP 2 018 867 A1**
(43) Veröffentlichungstag der Anmeldung: **28.01.2009**
(21) Anmeldenummer: 07014686.5
(22) Anmeldetag: 26.07.2007
(51) Int. Cl.: A61K 33/38, A61K 8/19, A61Q 11/00, A61Q 17/00

(54) **Verfahren zur Herstellung einer antimikrobiell wirkender kosmetischen und/oder pharmazeutischen Zusammensetzung zur topischen Anwendung**

(71) Anmelder: Spirig Pharma AG, CH-4622 Egerkingen (CH)
(72) Erfinder: Cichos, Christoph, 01468, Moritzburg (DE); Cichos, Irmgard, 01468, Moritzburg (DE)
(74) Vertreter: Bohest AG

(57) **Zusammenfassung**

Zur Herstellung einer antimikrobiell wirkenden kosmetischen oder pharmazeutischen Zusammensetzung wird zunächst Silberorthophosphat in photostabiler Form erzeugt und dieses dann in eine Zusammensetzung zur topischen Anwendung eingearbeitet.

## Beschreibung

Die Erfindung betrifft ein Verfahren zur Herstellung einer antimikrobiell wirkenden kosmetischen und/oder pharmazeutischen Zusammensetzung zur topischen Anwendung sowie die durch das Verfahren erhältliche Zusammensetzung. Die antimikrobielle Wirksamkeit der erfindungsgemäßen Zusammensetzung beruht auf der Freisetzung von Silberionen.

Der Einsatz von metallischem Silber und von Silberverbindungen zur Behandlung von Haut- und Schleimhauterkrankungen ist im Stand der Technik bekannt.

Die WO 90/08470 offenbart eine medizinische Substanz für topische Anwendungen. Die Substanz umfasst ein wasserlösliches Glas, welches elementares Silber oder eine Silberverbindung enthält. Bevorzugt ist eine Substanz, die als Silberverbindung Silberoxid und als Bestandteil des wasserlöslichen Glases Phosphorpentoxid enthält. Die Substanz wird zur Behandlung von Wunden und anderen topischen Infektionsstellen verwendet. Als Beispiele sind Eintrittsstellen von Kathetern und Kanülen genannt.

In der DE 103 42 258 A1 wird ein antimikrobiell wirkendes Präparat zur äußerlichen Anwendung für pharmazeutische oder kosmetische Zwecke beschrieben. Das Präparat wird unter Verwendung von nanoskaligem elementaren Silber hergestellt, welches auf einem inerten Trägermaterial abgeschieden ist. Als inertes Trägermaterial wird feinstteiliges Bariumsulfat genannt. Die Verwendung eines Trägermaterials, welches selbst ein Wirkstoff ist, wird durch die Lehre der DE 103 42 258 A1 ausdrücklich ausgeschlossen.

Die EP 0 251 783 offenbart antimikrobiell wirkende Zusammensetzungen, die unter anderem auch für topische Anwendungen vorgesehen sind. Die Zusammensetzungen enthalten eine antimikrobielle Silberverbindung auf einem Träger aus einem physiologisch inerten synthetischen Oxidmaterial. Bevorzugt wird Silberchlorid auf einem Träger aus Titandioxid. Auch Silbersulfat auf Titandioxid-Träger und Silberphosphat auf Titandioxid-Träger werden genannt. Es wird in der EP 0 251 783 A2 ausdrücklich angesprochen, dass es ein Nachteil von Zusammensetzungen sei, die Silberverbindungen enthielten, dass das ionische Silber in Gegenwart von Licht oder Strahlung instabil sei und zu metallischem Silber reduziert werde. Nach der Lehre der EP 0 251 783 A2 soll die unerwünschte Reduktion zu metallischem Silber und die damit verbundene Farbverdunkelung der Zusammensetzung dadurch verhindert werden, dass die Silberverbindung auf einen Träger aus einem physiologisch inerten synthetischen Oxidmaterial aufgebracht wird. Besonders gut soll die Photostabilisierung von Silberchlorid mit Hilfe eines Trägers aus Titandioxid gelingen. Zu Silberphosphat ist in der EP 0 251 783 A2 angegeben, dass sich dessen Instabilität in Gegenwart von Licht mit Hilfe eines Trägers weniger gut unterdrücken lässt.

In der EP 0 723 773 A1 ist ein Zahnreinigungsmittel beschrieben, welches ein calciumhaltiges Phosphat und ein antibakteriell wirksames Metall umfasst, welches in metallischem Zustand auf dem calciumhaltigen Phosphat geträgert ist. Das antibakteriell wirkende Metall ist Silber, Kupfer oder Zink. Die Herstellung umfasst die Reduktion zu dem antibakteriell wirkenden Metall auf dem Träger in einer Wasserstoffatmosphäre bei einer Temperatur von 350°C oder höher. Das Zahnreinigungsmittel ist gegen verschiedene Bakterienstämme wirksam.

Die WO 96/40171 offenbart eine Methode zur chemischen Erzeugung von Nekrosen an der Gebärmutterschleimhaut mit Silbernitrat-Dextran-Pasten. Wichtig ist, dass nach der erfolgreichen Behandlung ein deaktivierendes Reagenz, z.B. NaCl, zugeführt werden muss, um toxische Wirkungen des überschüssigen Silbers auszuschließen.

Neben der pharmazeutischen Verwendung von Silbernitrat ist auch die pharmazeutische Verwendung von anderen Silberverbindungen bekannt. So sind z.B. auch Silbersulphadiazin und Silberallantoinat zur Erzielung einer antimikrobiellen Wirkung verwendet worden.

Bei der topischen Anwendung einer leicht wasserlöslichen Silberverbindung wie Silbernitrat besteht die Gefahr der Überdosierung. Eine hohe Konzentration an Silberionen kann zytotoxische Wirkungen haben.

Grundsätzlich ist es auch möglich, in einer kosmetischen oder pharmazeutischen Zusammensetzung metallisches Silber in kollodider Form zu verwenden. Die antimikrobiell wirksame Spezies ist aber nicht das metallische Silber, sondern das Silberkation. Das Silber muss also oxidiert werden, um antimikrobielle Wirksamkeit entfalten zu können. Zwar wird die Oxidation erleichtert, wenn man Silberteilchen mit Größen im Nanometerbereich bereitstellt. Andererseits kann die Reduktion aber durch reduzierende Komponenten in der kosmetischen oder pharmazeutischen Zusammensetzung behindert werden. Dafür sind relativ schwache organische Reduktionsmittel wie z.B. Polyole ausreichend.

Präparate mit antimikrobiellen Metallen (Silber, Gold, Platin, Palladium, bevorzugt Silber) mit atomistischen Störungen werden in der WO 02/085387 und der WO 02/09729 zur Behandlung von entzündlichen Hauterkrankungen wie Ekzemen aller Art, Rosacea, Onychomycosis, Akne usw. vorgeschlagen. Die nanokristallinen Metalle werden als dünne Schichten auf Trägern, als Pulver oder Bestandteile von Pulvern oder suspendiert in Flüssigkeiten verwendet. Erzeugt werden die nanokristallinen Metalle durch Dampfabscheidung (DVP) auf geeignete Unterlagen unter Bedingungen, bei denen atomistische Störungen entstehen (WO 93/23902, WO 95/13704, WO 98/41095). Zur Herstellung eines Pulvers wird die Schicht von der Unterlage abgekratzt. Suspensionen von nanokristallinem Silber erhält man, indem man Silberpulver oder mit Silber beschichtete Materialien in einer geeigneten Flüssigkeit suspendiert. Ursache für die antimikrobielle Wirksamkeit dieser Präparate mit einem Gehalt an nanokristallinem Silber soll die Freisetzung von Ag⁰ oder Ag⁺/Ag⁰-Clustern im Kontakt mit flüssigen Phasen sein.

In Band 5 der 9. Auflage des Römpp Chemielexikon, Stuttgart 1992, ist Silberphosphat als gelbes, geruchloses Pulver beschrieben, bei dem am Licht allmählich Schwärzungen eintreten. Gemäß dem in diesem Handbuch wiedergegebenen allgemeinen Fachwissen ist Silberphosphat nicht photostabil. Im Hinblick auf die Verwendung in kosmetischen und pharmazeutischen Erzeugnissen ist dies ein Nachteil. Eine Creme, die sich nach dem Auftragen auf die Haut am Licht verfärbt, ist für den Verbraucher oder Patienten nicht akzeptabel.

Zur Überwindung der genannten Nachteile ist es Aufgabe der Erfindung, eine antimikrobiell wirkende kosmetische und/oder pharmazeutische Zusammensetzung zur topischen Anwendung zu schaffen, welche Silberionen in einer Menge freisetzt, die einerseits ausreicht um antibakteriell wirksam zu sein, und andererseits nicht toxisch ist, und die darüber hinaus photostabil ist. Zur Aufgabe gehört auch die Bereitstellung einer photostabilen kosmetischen und/oder pharmazeutischen Zusammensetzung zur topischen Anwendung, welche Silberionen in einer Konzentration freisetzt, die einerseits für die antimikrobielle Wirksamkeit ausreichen und andererseits aber nicht toxisch ist.

Gemäß einer ersten Ausführungsform der Erfindung wird die Aufgabe durch ein Verfahren zur Herstellung einer antimikrobiell wirkenden kosmetischen und/oder pharmazeutischen Zusammensetzung zur topischen Anwendung gelöst, umfassend
(a) Bereitstellung einer Lösung eines Silbersalzes in wässrigem Ammoniak,
(b) Zugabe von Phosphorsäure unter Ausfällung von Silberorthophosphat als Feststoff, der in Suspension erhalten wird, bis der pH-Wert der Suspension zwischen 6,0 und 8,0 liegt,
(c) Abtrennen des Feststoffes,
(d) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 50 µS/cm aufweist;
(e) Trocknen des Feststoffes und
(f) Einarbeiten des Feststoffes in eine Zusammensetzung zur topischen Anwendung.

Gemäß einer zweiten Ausführungsform der Erfindung wir die Aufgabe durch ein Verfahren zur Herstellung einer antimikrobiell wirkenden kosmetischen und/oder pharmazeutischen Zusammensetzung zur topischen Anwendung gelöst, umfassend
(a) Bereitstellung einer Lösung eines Silbersalzes in wäßrigem Ammoniak,
(b) Zugabe von Phosphorsäure unter Ausfällung von Silberorthophosphat als Feststoff, der in Suspension enthalten wird, bis der pH-Wert der Suspension zwischen 6,0 und 8,0 liegt,
(c) Abtrennen des Feststoffes,
(d) Suspendieren des Feststoffes in deionisiertem Wasser,
(e) Zugabe von wässrigem Ammoniak zu der Suspension und Erwärmung der Mischung auf eine Temperatur im Bereich von 40-90°C, wobei die Menge an Ammoniak so bemessen ist, dass bei der gewählten Temperatur im Bereich von 40-90°C 1 Gew.% bis 80 Gew.% der Silberionen in Lösung gehen,
(f) Zugabe eines Reduktionsmittels bei der gewählten Temperatur im Bereich von 40-90°C in einer Menge, die ausreicht, die in Lösung befindlichen Silberionen zu metallischem Silber zu reduzieren, so dass ein teilreduziertes Silberorthophosphat als suspendierter Feststoff gebildet wird,
(g) Abtrennen des Feststoffes,
(h) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 20 µS/cm aufweist,
(i) Trocknen des Feststoffes,
(j) Einarbeiten des Feststoffes in eine Zusammensetzung zur topischen Anwendung.

Gemäß einer dritten Ausführungsform der Erfindung wird die Aufgabe durch ein Verfahren zur Herstellung einer antimikrobiell wirkenden kosmetischen und/oder pharmazeutischen Zusammensetzung zur topischen Anwendung gelöst, umfassend
(a) Bereitstellung einer Lösung eines Silbersalzes in wässrigem Ammoniak,
(b) Zugabe von Phosphorsäure unter Ausfällung von Silberorthophosphat als Feststoff, der in Suspension erhalten wird, bis der pH-Wert der Suspension zwischen 6,0 und 8,0 liegt,
(c) Abtrennen des Feststoffes,
(d) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 50 µS/cm aufweist,
(e) Suspendieren des Feststoffes in deionisiertem Wasser,
(f) Zugabe von wässrigem Ammoniak zur Suspension und Erwärmung der Mischung auf eine Temperatur im Bereich von 40-90°C, wobei die Menge an Ammoniak so bemessen ist, dass bei der gewählten Temperatur im Bereich von 40-90°C 1 Gew.% bis 80 Gew.% der Silberionen in Lösung gehen,
(g) Zugabe eines Reduktionsmittels bei der gewählten Temperatur im Bereich von 40-90°C in einer Menge, die ausreicht, die in Lösung befindlichen Silberionen zu metallischem Silber zu reduzieren, so dass ein teilreduziertes Silberorthophosphat als Feststoff gebildet wird,
(h) Abtrennen des Feststoffes,
(i) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 20 µS/cm aufweist,
(j) Trocknen des Feststoffes,
(k) Einarbeiten des Feststoffes in eine Zusammensetzung zur topischen Anwendung.

Überraschenderweise wird durch das erfindungsgemäße Verfahren eine Zusammensetzung erhalten, die trotz ihres Gehaltes an Silberphosphat photostabil ist. Darüber hinaus hat die Verwendung von Silberphosphat in einer kosmetischen oder pharmazeutischen Zusammensetzung den großen Vorteil, dass es sich um eine in Wasser schwer lösliche Verbindung handelt. Die Löslichkeit ist zwar groß genug, um Silberionen in einer Menge freizusetzen, dass die erwünschte antimikrobielle Wirksamkeit erzielt wird. Die Wasserlöslichkeit von Silberphosphat ist aber andererseits so gering, dass zytotoxische Wirkungen ausgeschlossen werden können.

Neben bzw. aufgrund der antibakteriellen Wirksamkeit haben die Silberionen auch eine stabilisierende Wirkung. Das erfindungsgemäße Verfahren eignet sich daher auch zur Langzeitstabilisierung von kosmetischen und/oder pharmazeutischen Zusammensetzungen. Zur Stabilisierung der Zusammensetzung ist im Regelfall eine geringere Silberkonzentration erforderlich als für eine therapeutische Verwendung. Die Erfindung bezieht sich auch auf kosmetische Zusammensetzungen, die lediglich durch einen Gehalt an Silberorthophosphat oder teilreduziertem Silberorthophosphat stabilisiert sind und die keine therapeutische Wirkung im medizinischen Sinne haben.

Die Zusammensetzung gemäß der ersten Ausführungsform enthält Silberorthophosphat. Die Zusammensetzung gemäß der zweiten und dritten Ausführungsform enthält teilreduziertes Silberorthophosphat. Die zweite und die dritte Ausführungsform des erfindungsgemäßen Verfahrens unterscheiden sich nur dadurch, dass bei der dritten Ausführungsform das Silberorthophosphat vor der Teilreduktion durch wiederholtes Waschen gereinigt wird.
Durch diese vorgezogene Reinigung vereinfachen sich die weiteren Verfahrensschritte und insbesondere die abschließende Reinigung des teilreduzierten Silberorthophosphates.

Im Rahmen der vorliegenden Erfindung wird der Begriff "Feststoff" als Oberbegriff für die Bezeichnung von Silberorthophosphat und teilreduziertem Silberorthophosphat verwendet. Für andere feste Materialien, die weitere Bestandteile der erfindungsgemäßen Zusammensetzung sein können, werden andere Begriffe verwendet, beispielsweise der Begriff "Füllstoff". Der Begriff "Feststoff" bezieht sich also nicht auf derartige weitere Bestandteile oder Zusatzstoffe, es sei denn, dass ausdrücklich etwas anderes angegeben ist.

Die erfindungsgemäß erzielte Verbesserung der Photostabilität wird erzielt durch wiederholtes Waschen des Silberorthophosphates oder des teilreduzierten Silberorthophosphates, bis die spezifische Leitfähigkeit der letzten Probe Waschwasser nach der Abtrennung von dem Feststoff einen Wert unter 50 µS/cm im Falle des Silberorthophosphates oder einen Wert unter 29 µS/cm im Falle des teilreduzierten Silberphosphates aufweist. Es ist überraschend, dass sich eine befriedigende Photostabilität mit Hilfe eines derart kontrollierten Waschvorganges erzielen lässt.

Ein weiterer wesentlicher Vorteil ist darin zu sehen, dass es bei dem erfindungsgemäßen Verfahren nicht erforderlich ist, zur Erzielung der Photostabilität das Silberphosphat auf ein anorganisches Trägermaterial aufzubringen. Vielmehr ist es erfindungsgemäß besonders bevorzugt, dass das Silberphosphat oder das teilreduzierte Silberphosphat nicht auf ein anorganisches Trägermaterial und insbesondere nicht auf ein physiologisch inertes anorganisches Oxidmaterial wie Titandioxid oder allgemeiner Oxide des Titans, Magnesiums, Aluminiums, Siliziums, Cers, Zirconiums, Hafniums, Niobs und Tantals oder auf Calciumhydroxylapatit oder Bariumsulfat aufgebracht wird. Dementsprechend sind die erfindungsgemäßen Zusammensetzungen vorzugsweise frei von einem anorganischen Trägermaterial für das Silberphosphat oder das teilreduzierte Silberphosphat und insbesondere frei von den vorstehend genannten Trägermaterialien. Ganz besonders ist es bevorzugt, dass bei dem erfindungsgemäßen Verfahren die vorstehend genannten Trägermaterialien überhaupt nicht verwendet werden, also weder als Trägermaterial noch als Zusatz wie beispielsweise als Füllstoff. Demgemäß sind ganz besonders bevorzugte erfindungsgemäße Zusammensetzungen frei von Oxiden des Titans, Magnesiums, Aluminiums, Siliciums, Cers, Zirconiums, Hafniums, Niobs und Tantals sowie von Calciumhydroxylapatit und Bariumsulfat.

Gemäß aller Ausführungsformen der Erfindung wird zunächst eine Lösung eines Silbersalzes in wässrigem Ammoniak hergestellt. Zu diesem Zweck kann ein beliebiges Silbersalz mit ausreichender Wasserlöslichkeit verwendet werden. Ein bevorzugtes Silbersalz ist Silbernitrat.

Aus dieser Lösung wird Silberorthophosphat durch Zugabe von Phosphorsäure ausgefällt. Diese Ausfällung wird in dem Fachmann bekannter Weise so ausgeführt, dass das Silberorthophosphat in Suspension erhalten wird. Die suspendierten Feststoffteilchen aus Silberorthophosphat haben die erfindungsgemäß erwünschte geringe Teilchengröße. Aus den kleinen Teilchen können die Silberionen in der kosmetischen oder pharmazeutischen Zusammensetzung besonders gut freigesetzt werden. Phosphorsäure wird zur Ausfällung des Silberphosphats so lange zugegeben, bis der pH-Wert der Suspension zwischen 6,0 und 8,0 liegt.

Das Silberorthophosphat wird dann aus der Suspension abgetrennt. Die Abtrennung erfolgt vorzugsweise durch Zentrifugieren.

Gemäß der ersten Ausführungsform der Erfindung wird das Silberorthophosphat anschließend wiederholt mit Portionen von deionisiertem Wasser gewaschen. Zu diesem Zweck wird das Silberorthophosphat vorzugsweise für jeden Waschvorgang zunächst in Reinstwasser dispergiert und anschließend durch Zentrifugieren wieder abgetrennt. Die Waschvorgänge werden so oft wiederholt, bis die spezifische Leitfähigkeit des Waschwassers nach Abtrennung von dem Feststoff einen Wert unter 50 µS/cm aufweist.

Die spezifische elektrische Leitfähigkeit ist das Reziproke des spezifischen Widerstands. Die Einheit ist (Ω/cm)⁻¹ bzw. S/cm. Hierbei ist S = 1/Ω die Kurzform für Siemens, die Einheit des elektrischen Leitwertes, welches definiert ist als das Reziproke des elektrischen Widerstandes. Die elektrische Leitfähigkeit hängt von der Temperatur ab sowie bei Ionenleitern von der Konzentration, dem Dissoziationsgrad und dem Lösungsmittel. Für die Zwecke der vorliegenden Erfindung bezieht sich die elektrische Leitfähigkeit auf die Referenztemperatur 25°C.

Leitfähigkeitsmessgeräte werden auch als Konduktometer bezeichnet. Erfindungsgemäß geeignete Konduktometer sind unter den Bezeichnungen Sartorius PP-20 und Sartorius PP-50 im Handel erhältlich. Hersteller ist die Sartorius AG, Weender Landstraße 94-108, Göttingen, Deutschland. Die von der Sartorius AG für diese Konduktometer angebotenen Messzellen PY-C01 - PY-C03 sind 4-Elektroden-Messzellen mit Platinelektroden. Die Leitfähigkeitsmessung wird mit diesen Konduktometern unter Anwendung einer Wechselspannung bzw. eines Wechselstromes durchgeführt. Die gebräuchlichste Frequenz ist 50 Hz.

Das durch wiederholtes Waschen ausreichend gereinigte Silberorthophosphat wird anschließend getrocknet. Das Trocknen kann in beliebiger Weise erfolgen, beispielsweise in einem Trockenschrank. Das so erhaltene getrocknete Silberorthophosphat ist dann geeignet für die Einarbeitung in eine kosmetische und/oder pharmazeutische Zusammensetzung.

Gemäß der zweiten Ausführungsform der Erfindung wird wie bei der ersten Ausführungsform zunächst eine Suspension von Silberorthophosphat hergestellt. Das Silberorthophosphat wird dann abgetrennt und in deionisiertem Wasser erneut suspendiert. Anschließend wird wässriger Ammoniak zu der Suspension gegeben. Die Mischung wird auf eine Temperatur im Bereich von 40-90°C erwärmt. Die Menge an Ammoniak wird dabei so bemessen, dass bei der gewählten Temperatur im Bereich von 40-90°C eine Menge von 1 Gew.% bis 80 Gew.% des in der Mischung vorhandenen Silbers bzw. der in der Mischung vorhandenen Silberionen in Lösung gehen.

Durch den Übergang in Lösung sollen die Silberionen in größerer Menge für den anschließenden Reduktionsschritt verfügbar gemacht werden. Für die Reduktion stehen dann einerseits die in Lösung gebrachten Silberionen zur Verfügung. Andererseits kann die Reduktion natürlich auch an der Oberfläche der suspendierten Teilchen stattfinden.

Das Reduktionsmittel wird bei der gewählten Temperatur im Bereich von 40-90°C in einer Menge zugegeben, die ausreicht, mindestens die in Lösung befindlichen Silberionen zu metallischem Silber zu reduzieren. Natürlich kann man einen gewissen Überschuss an Reduktionsmittel einsetzen. Vorzugsweise ist die Menge an Reduktionsmittel groß genug, um sowohl die in Lösung befindlichen Silberionen als auch die an der Oberfläche der suspendierten Teilchen befindlichen Silberionen zu reduzieren.

Über den Einsatz von Ammoniak und Reduktionsmittel lässt sich die erzeugte Menge an metallischem Silber steuern. Gleichzeitig wird damit die Färbung des erzeugten Feststoffes eingestellt.

Es wird angenommen, dass bei dem Verfahren gemäß der zweiten und dritten Ausführungsform der Erfindung metallische Silberteilchen im nanoskaligen Bereich auf Silberphosphat abgeschieden werden. Dadurch entstehen viele mikroskopische elektrochemische Halbelemente Ag/Ag₃PO₄, deren Potenziale nur von der Phosphationenkonzentration in der umgebenden wässrigen Phase abhängen. Thermodynamisch lässt sich zeigen, dass dadurch die freie Enthalpie für die Reaktion Ag⁰ → Ag⁺ + e in den negativen Bereich verschoben wird und damit die Reduktion von Silberionen zu Silber erschwert wird.

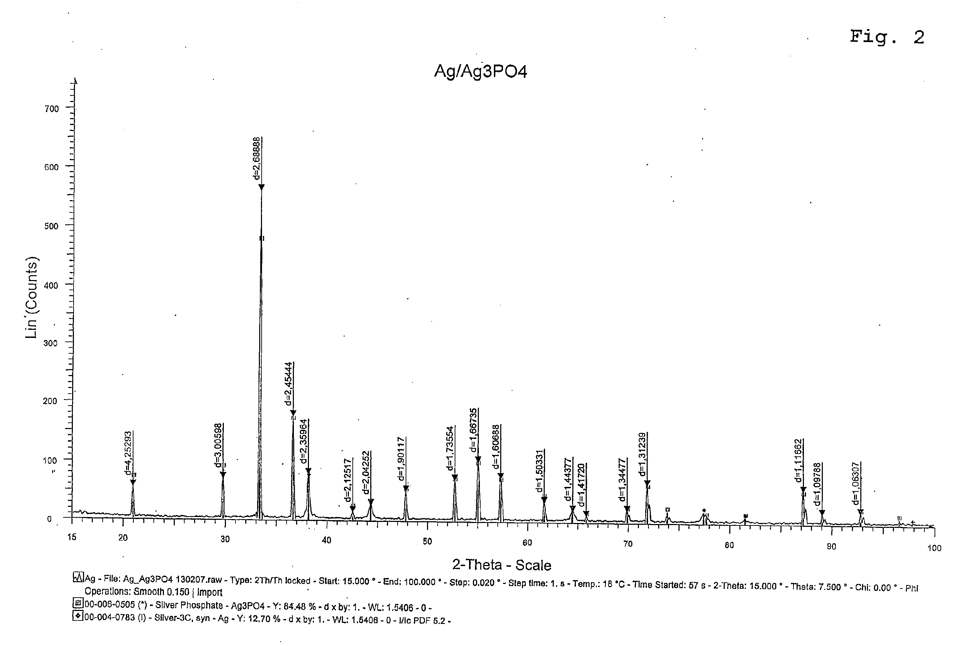

Das Produkt der Reduktion wird als teilreduziertes Silberorthophosphat bezeichnet. Die Figur 2 zeigt ein Röntgenspektrum eines solchen teilreduzierten Silberorthophosphates. In dem Röntgenspektrum sind sowohl Peaks für Silberorthophosphat als auch Banden für metallisches Silber zu erkennen.

Für den Reduktionsschritt können verschiedene Reduktionsmittel verwendet werden. Bevorzugte Reduktionsmittel sind verschiedene Zucker, Aldehyde, Hydrochinon, Weinsäure und Hydrazin. Ein besonders bevorzugtes Reduktionsmittel ist Glucose.

Wie bereits erwähnt, besteht zwischen der zweiten und der dritten Ausführungsform der Erfindung nur ein geringer Unterschied. Gemäß der dritten Ausführungsform wird das Silberorthophosphat vor der Teilreduktion so lange gewaschen, bis es dem Reinheitskriterium gemäß der ersten Ausführungsform genügt, d.h. bis es eine Leitfähigkeit unter 50 µS/cm aufweist.

Wie auch bereits angesprochen, soll die Menge an Reduktionsmittel so bemessen sein, dass die in Lösung befindlichen Silberionen zu metallischem Silber reduziert werden. Dennoch können unter Umständen geringe Mengen an Silberionen in der Lösung verbleiben. Deshalb ist es bevorzugt, dass vor dem Abtrennen des teilreduzierten Orthophosphates der Suspension Phosphorsäure zugesetzt wird, um einen pH-Wert im Bereich von 6,0 bis 8,0 einzustellen. Dadurch werden eventuell in Lösung befindliche Silberionen in Silberorthophosphat überführt, das aus der Lösung in Suspension ausgefällt wird.

Die Reinigung des teilreduzierten Silberorthophosphats erfolgt genauso durch wiederholtes Waschen wie die Reinigung des reinen Silberorthophosphats. Allerdings.ist erfindungsgemäß vorgesehen, dass die Leitfähigkeit der letzten Portion Waschwasser bei teilreduziertem Silberorthophosphat weniger als 20 µS/cm beträgt.

Bei dem erfindungsgemäßem Verfahren wird vorzugsweise ausschließlich Reinstwasser verwendet. Reinstwasser kann beispielsweise durch eine Destillation über eine mehrbödige Kolonne erhalten werden. Reinstwasser kann aber auch durch Deionisation erhalten werden. Die Leitfähigkeit des Reinstwassers ist sehr gering und liegt in jedem Fall unter 5 µS/cm.

Das Silberorthophosphat oder das teilreduzierte Silberorthophosphat wird schließlich in die kosmetische oder pharmazeutische Zusammensetzung eingearbeitet, d.h., es wird mit den übrigen Bestandteilen der Zusammensetzung vermischt. Die übrigen Bestandteile werden in dem Fachmann bekannter Weise in Abhängigkeit von der topischen Applikationsform ausgewählt, d.h. in Abhängigkeit davon, ob eine Salbe, eine Creme, eine Lotion, ein Gel, eine Zahnpasta usw. hergestellt werden soll.

Man kann so vorgehen, dass man zunächst eine Grundzusammensetzung herstellt, beispielsweise eine Salbengrundlage. Man kann auch eine im Handel erhältliche Salbengrundlage verwenden. Das Silberorthophosphat oder das teilreduzierte Silberorthophosphat wird dann in die Salbengrundlage eingemischt.

Alternativ kann man aber auch so vorgehen, dass man zunächst eine Vormischung aus dem Silberorthophosphat oder dem teilreduzierten Silberorthophosphat mit mindestens einem weiteren Bestandteil der vorgesehenen Zusammensetzung herstellt. Diese Vormischung wird dann durch Vermischen mit den weiteren Bestandteilen zu der fertigen Zusammensetzung zur topischen Anwendung weiter verarbeitet.

Das Silberorthophosphat oder das teilreduzierte Silberorthophosphat werden vorzugsweise in einer Menge von 0,001 Gew.% bis 10 Gew.%, insbesondere 0,05 Gew.% bis 4 Gew.%, bezogen auf das Gesamtgewicht der gebrauchsfertigen Zusammensetzung zur topischen Anwendung, in die Zusammensetzung eingearbeitet.

Die erfindungsgemäßen kosmetischen oder pharmazeutischen Präparate sind neu aufgrund ihrer Zusammensetzung und aufgrund der Photostabilität bei dieser Zusammensetzung.

Die erfindungsgemäße Zusammensetzung zur topischen Anwendung kann eine Salbe, eine Creme, eine Lotion, ein Gel, eine Zahnpasta oder auch ein Deodorant sein. Das Deodorant kann insbesondere in der Form eines Deodorantstiftes vorliegen, in welchem das Silberorthophosphat oder das teilreduzierte Silberorthophosphat dispergiert ist.

Silberionen wirken bei Erkrankungen, die durch Bakterien, durch Viren oder durch Pilze hervorgerufen werden, also bei Mucosen.

Die erfindungsgemäße Zahnpasta eignet sich zur Bekämpfung der Plaque- und Zahnsteinbildung und zur Vorbeugung gegen und Behandlung der Paradontose.

Mit der erfindungsgemäßen Zusammensetzung kann Akne vulgaris behandelt werden. Auch Ekzeme lassen sich erfolgreich behandeln.

Mit der erfindungsgemäßen Zusammensetzung können Hauterkrankungen behandelt werden, die durch Herpesviren wie Herpes simplex hervorgerufen werden.

Auch Pilzerkrankungen wie beispielsweise Fußpilz können mit der erfindungsgemäßen Zusammensetzung behandelt werden.

Die erfindungsgemäße Zusammensetzung ist gut geeignet zur Behandlung von Wundinfektionen aller Art. Alle in der WO 90/08470 genannten Einsatzbereiche kommen auch für die Zusammensetzung gemäß der vorliegenden Erfindung in Betracht. Insbesondere kann man die erfindungsgemäße Zusammensetzung verwenden, um Entzündungen an Eintrittsstellen von Kathetern und Kanülen vorzubeugen bzw. diese zu behandeln.

In Form eines Nasensprays kann die erfindungsgemäße pharmazeutische Zusammensetzung auch zur Behandlung von Schnupfen verwendet werden.

MRSA ist die Abkürzung für Methicillin-resistenter Staphylococcus aureus und bezeichnet gegen bestimmte Antibiotika resistente Stämme von Staphylococcus aureus. Methicillin ist - wie das Penicillin - ein β-Lactan-Antibiotikum, das als Testantibiotikum für die Antibiotika-Sensibilität von Bakterien benutzt wird. Ist ein Bakterium resistent gegen Methicillin, ist es normalerweise resistent gegen alle β-Lactan-Antibiotika. Die erfindungsgemäße pharmazeutische Zusammensetzung kann zur Behandlung von Haut- und Schleimhauterkrankungen eingesetzt werden, die durch MRSA hervorgerufen werden.

Die Erfindung wird nachfolgend anhand von Beispielen näher erläutert.

### Beispiel 1 - Herstellung von Silberorthophosphat

Es wurde ein Dreihalskolben mit Glasrührer in einem Wasserbad verwendet. In dem Kolben wurden 700 ml Reinstwasser (deionisiertes Wasser) vorgelegt und darin 121,7 g AgNO₃ gelöst. Der Lösung wurden 120 ml einer 25%igen NH₃-Lösung zugefügt. Der anfängliche braune Niederschlag wurde dabei wieder vollständig aufgelöst.

Der Kolbeninhalt wurde auf 60°C erwärmt. Nach dem Erreichen der Endtemperatur wurde begonnen, langsam 200 ml einer 4-molaren H₃PO₄-Lösung aus einem Tropftrichter zuzutropfen. Es bildete sich an der Eintropfstelle ein gelblicher Niederschlag, der sich zunächst wieder auflöste, mit abnehmendem pH-Wert aber beständig blieb.

Nach Abschluss der Phosphorsäurezugabe wurde der pH-Wert gemessen. Er lag innerhalb des Bereiches von 6,0 bis 6,8. Durch Zugabe eines Tropfens Phosphorsäure in das klare Filtrat wurde gezeigt, dass die Fällung vollständig war.

Nach dem Erkalten wurde aus der so erhaltenen Suspension der Feststoff durch Zentrifugieren abgetrennt. Anschließend wurde der Feststoff wieder in einem großen Volumen deionisiertem Wasser dispergiert. Aus dieser wässrigen Suspension wurde der Feststoff erneut abgetrennt. Dieser Waschvorgang wurde mehrfach wiederholt. Das letzte Waschfiltrat wies eine elektrische Leitfähigkeit von weniger als 50 µS/cm auf.

Anschließend wurde der gewaschene Feststoff bei 80 bis 90°C in einem Umlufttrockenschrank getrocknet.

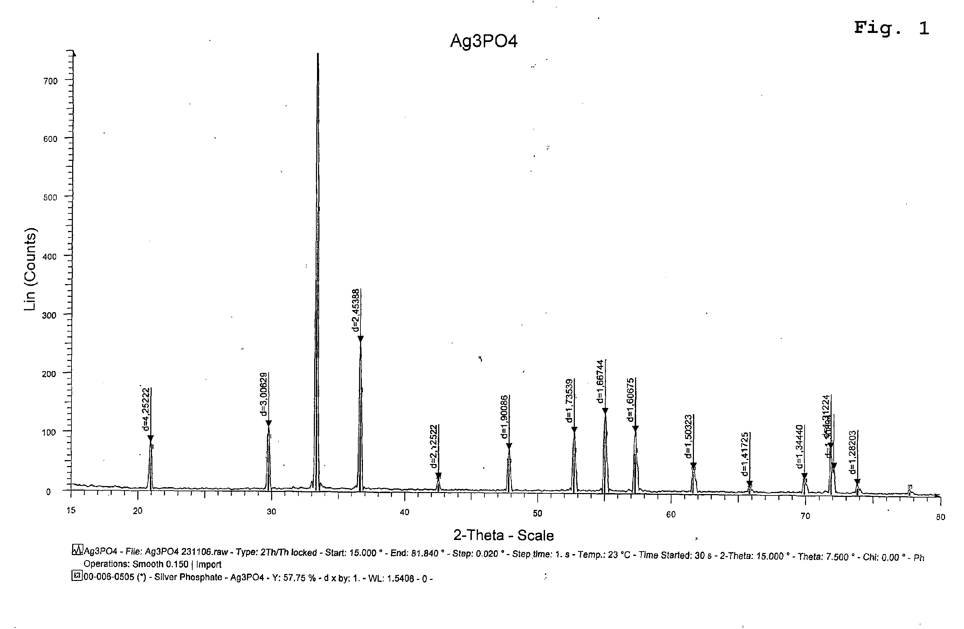

Eine Röntgendiffraktionsaufnahme des erhaltenen Feststoffs ist in Figur 1 gezeigt. Es handelt sich um reines kristallines Silberorthophosphat.

### Beispiel 2 - Prüfung der Lichtbeständigkeit

Das nach Beispiel 1 hergestellte Silberorthophosphat wurde sowohl in trockener Form als auch in einer Suspension in gewöhnlichem Leitungswasser in Glasgefäßen bzw. PE-Gefäßen über 8 Wochen im Freien dem Tageslicht ausgesetzt. Es wurde keine Farbveränderung beobachtet.

Proben von reinem Silberorthophosphat und Proben von 10% Silberorthophosphat auf Bariumsulfat wurden belichtet und unbelichtet verglichen. Dazu wurden der Gesamtsilbergehalt und der Gehalt an ionischem Silber in den Proben analytisch gemessen. Die belichteten Proben waren einer Belichtung durch Tageslicht über 8 Wochen im Freien ausgesetzt. Die Ergebnisse sind in Tabelle 1 wiedergegeben:

**Tabelle 1 - Analytische Silbergehalte vor und nach Belichtung der Proben**

| Probe | Nach Belichtung | | Ohne Belichtung | |
|---|---|---|---|---|
| | Ag-gesamt/% | Ag⁺/% | Ag-gesamt/% | Ag⁺/% |
| Ag₃PO₄ | 78,89 +/- 1,41 | 78,83 +/- 1,61 | 80,58 +/- | 5,96 78,38 +/- 0,08 |
| 10% Ag₃PO₄ auf BaSO₄ | 7,67 +/- 0,10 | 7,92 +/- 0,20 | 7,62 +/- 0,07 | 7,80 +/- 0,08 |

Die in der Tabelle angegebenen Fehlerbereiche sind aus Vielfachmessungen errechnet. Der theoretische Silbergehalt in Ag₃PO₄ beträgt 77,31 Gew.%.

Wird dem Leitungswasser für die Suspendierung des Silberorthophosphates Glucose als Reduktionsmittel zugesetzt, so ist bereits nach zwei Stunden Belichtung die erwartete oberflächliche Reduktion in einer Farbverschiebung nach grün zu beobachten. Eine Möglichkeit, solchen überraschenden Farbveränderungen in kosmetischen oder pharmazeutischen Zusammensetzungen, die reduzierende Substanzen enthalten, vorzubeugen, wurde in der Teilreduktion des reinen Silberorthophosphates gemäß der zweiten und dritten Ausführungsform der Erfindung gefunden.

### Beispiel 3 - Teilreduktion von Ag₃PO₄

5 g Ag₃PO₄ wurden in 150 ml Reinstwasser suspendiert. Bei dem Ag₃PO₄ handelte es sich um getrocknetes Material, das nach Verfahren gemäß Beispiel 1 hergestellt worden war. Nach Zugabe von 1,7 ml 25%iger NH₃-Lösung wurde die Suspension auf 60°C erwärmt. 1,1 g Glucosemonohydrat wurden in 150 ml Reinstwasser gelöst, in einen Tropftrichter überführt und nach Erreichen der Temperatur von 60°C langsam zu der Suspension zugetropft. Die Tropfzeit betrug zwischen 1,5 und 2 Stunden.

Nach Beendigung der Reduktion hatte sich eine blaugraue Suspension gebildet. Der pH-Wert lag zwischen 8 und 9. Eine Freisetzung von Ammoniak während der Reduktion war deutlich wahrzunehmen.

Der Suspension wurde Phosphorsäure zugegeben, um den pH-Wert auf einen Wert im Bereich von 6 bis 6,8 einzustellen. Dabei veränderte sich die Farbe von blaugrau nach grünbraun.

Der Feststoff wurde von der Flüssigkeit abgetrennt, erneut in Reinstwasser dispergiert und wieder abgetrennt. Das Abtrennen des Feststoffes erfolgte durch Zentrifugieren. Der Waschvorgang wurde viermal wiederholt. Die elektrische Leitfähigkeit des letzten Filtrates (Waschwassers) betrug <20 µS/cm. Anschließend wurde der Feststoff getrocknet.

Durch chemische Analyse der Zusammensetzung wurden folgende Werte ermittelt:
Ag⁺-Gehalt: 58,02 Gew.%
Ag°-Gehalt: 19,68 Gew.%

Eine Röntgendiffraktionsaufnahme dieses Produktes ist in Figur 2 gezeigt. Neben Peaks für Silberorthophosphat sind auch Banden für metallisches Silber zu erkennen.

### Beispiel 4 - Silberfreisetzung aus Silberorthophosphat bei verschiedenen pH-Werten

In drei Erlmeierkolben wurden jeweils 40 ml Reinstwasser vorgelegt. Die in der nachfolgenden Tabelle 2 angeführten Ausgangs-pH-Werte pH₀ würden eingestellt. Dann wurde jeweils 1 g Silberorthophosphat zugesetzt. Die Erlmeierkolben wurden verschlossen und drei Stunden bei Zimmertemperatur gesschüttelt. Danach wurden im klaren Filtrat jeweils der End-pH-Wert und die Silberkonzentration gemessen.

**Tabelle 2: Silberfreisetzung aus AG₃PO₄ bei verschiedenen pH-Werten**

| Vers.-Nr. | pH_{Anfang} | PH_{Ende} | Ag-Konz. mg/l |
|---|---|---|---|
| 1 | 9,15 | 7,44 | 6,8 |
| 2 | 3,77 | 7,40 | 26,4 |
| 3 | 5,20 | 7,40 | 10,0 |

Bei einem Ausgangs-pH-Wert von 3,77 wird eine verstärkte Löslichkeit beobachtet. (Ohne hydrolytischen Effekt sollte die Konzentration bei 5 mg/l liegen.)

Bei der Einarbeitung des Silberorthophosphats in eine kosmetische oder pharmazeutische Zusammensetzung ist dieser Umstand zu beachten. Ebenso ist zu beachten, dass Silberphosphat eine Pufferwirkung besitzt. Die Silberfreisetzung aus den erfindungsgemäßen kosmetischen oder pharmazeutischen Zusammensetzungen wird niedriger sein als in diesem Beispiel.

### Beispiel 5 - Zahnpasta

In eine käufliche Zahnpasta, die kein Triclosan enthielt, wurden 0,25 Gew.%, bezogen auf das Gewicht der käuflichen Zahnpasta, des Silberorthophosphates gemäß Beispiel 1 eingearbeitet.

Zum Vergleich wurde dieselbe käufliche Zahnpasta ohne Zusatz von Silberorthophosphat herangezogen.

Die beiden Zahnpasten wurden unter zahnärztlicher Kontrolle an Probanden qualitativ getestet. Nach 14-tägiger Anwendung der erfindungsgemäßen Zahnpasta wurden deutliche Verbesserungen beobachtet. Plaquebildung und Zahnfleischbluten waren nachweislich zurückgegangen. Bei den Probanden, welche die Zahnpasta ohne Zusatz von Silberorthophosphat benutzten, zeigte sich keine Wirkung.

Die Konzentrationsabhängigkeit der Wirkung des Silberorthophosphates in der Zahnpasta wurde untersucht. Zu diesem Zweck wurden in die käufliche Zahnpasta, die kein Triclosan enthielt, einmal 0,5 Gew.%, bezogen auf das Gewicht der käuflichen Zahnpasta, und einmal 0,1 Gew.%, bezogen auf das Gewicht der käuflichen Zahnpasta, eingearbeitet. Bei Probanden, welche die Zahnpasta mit 0,5 Gew.% Silberorthophosphat benutzten, war der Effekt größer als bei Probanden, welche die Zahnpasta mit 0,1 Gew.% Silberphosphat benutzten.

### Beispiel 6 - Fettcreme

In eine im Handel erhältliche, rezeptfreie Fettcreme mit ungesättigten Fettsäuren wurden 0,3 Gew.%, bezogen auf das Gewicht der im Handel erhältlichen Fettcreme, des Silberorthophosphates gemäß Beispiel 1 eingearbeitet.

Die so erhaltene erfindungsgemäße Creme wurde einem Probanden mit aknebedingten Pusteln zweimal täglich im Gesicht aufgetragen. Bereits nach einem Tag zeigte sich ein deutlicher Rückgang der Entzündung. Nach drei Tagen waren die Entzündungsherde abgeheilt.

Eine Farbveränderung der erfindungsgemäßen Fettcreme mit einem Gehalt an Silberphosphat bei der mehrwöchigen Aufbewahrung der Fettcreme in einem Glasgefäß bei Tageslicht war nicht zu beobachten.

In einem weiteren Versuch wurde der gleichen im Handel erhältlichen Fettcreme 0,3 Gew.%, bezogen auf das Gewicht der im Handel erhältlichen Fettcreme, des teilreduzierten Silberorthophosphats gemäß Beispiel 3 zugesetzt. Diese zweite erfindungsgemäße Fettcreme zeigte eine genauso gute Wirkung bei der Behandlung von aknebedingten Pusteln im Gesicht.

In kosmetischen und pharmazeutischen Zusammensetzungen wird bevorzugt reines Silberorthophosphat verwendet, das eine angenehmere Farbe aufweist. Wie dieses Beispiel zeigt, kann aber grundsätzlich auch teilreduziertes Silberorthophosphat eingesetzt werden.

### Beispiel 7 - Wirkung an Patientenisolat Staphylococcus aureus

Es wurde eine Zusammensetzung hergestellt, die aus 5 Gew.% Silberorthophosphat gemäß Beispiel 1 und 95 Gew.% Bariumsulphat (B/5P) bestand. Eine solche Zusammensetzung kann beispielsweise als Pulver auf entzündete Wunden aufgestreut werden.

Die antibakterielle Wirksamkeit dieser Zusammensetzung bei verschiedenen Dosierungen wurde an einem Patientenisolat Staphylococcus areus geprüft. Der Keimabbau in Abhängigkeit von der Zeit ist in der nachfolgenden Tabelle 3 gezeigt. Die Dosierung ist in Milligramm B/5P je Gramm Patientenisolat angegeben.

**Tabelle 3: Keimabbau in Abhängigkeit der Zeit Keime: Staph.aureus, Patientenisolat**

| Dosierung B/5P | CPU nach | | | |
|---|---|---|---|---|
| | 0 h | 6 h | 20 h | 24 h |
| 1 mg/g | 50 000 | 30 000 | 0 | 0 |
| 0,1 mg/g | 50 000 | 40 000 | 0 | 0 |
| 0,01 mg/g | 50 000 | 50 000 | 0 | 0 |
| 0,0001 mg/g | 50 000 | 50 000 | 50 000 | 50 000 |
| ohne | 50 000 | 50 000 | 50 000 | 50 000 |

Nach Einwirkzeiten zwischen 6 und 20 Stunden sind bei einer Dosierung von 0,01 mg oder höher alle Keime im Patientenisolat abgetötet. Dieser Befund verdeutlicht, dass für den Einsatz in pharmazeutischen Präparaten nur geringe Mengen erforderlich sind und die Wahrscheinlichkeit einer zytotoxischen Reaktion auszuschließen ist.

Vorzugsweise wird das in diesem Beispiel beschriebene Gemisch aus Silberorthophosphat und Bariumsulfat nicht als solches eingesetzt, sondern es stellt eine Vormischung für eine pharmazeutische Zusammensetzung dar, welche eine einfachere Dosierung erlaubt wie z.B. ein pharmazeutisches Gel.

## Patentansprüche

1. Verfahren zur Herstellung einer antimikrobiell wirkenden kosmetischen und/oder pharmazeutischen Zusammensetzung zur topischen Anwendung, umfassend
(g) Bereitstellung einer Lösung eines Silbersalzes in wässrigem Ammoniak,
(h) Zugabe von Phosphorsäure unter Ausfällung von Silberorthophosphat als Feststoff, der in Suspension erhalten wird, bis der pH-Wert der Suspension zwischen 6,0 und 8,0 liegt,
(i) Abtrennen des Feststoffes,
(j) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 50 µS/cm aufweist;
(k) Trocknen des Feststoffes und
(l) Einarbeiten des Feststoffes in eine Zusammensetzung zur topischen Anwendung.

2. Verfahren zur Herstellung einer antimikrobiell wirkenden kosmetischen und/oder pharmazeutischen Zusammensetzung zur topischen Anwendung, umfassend
(k) Bereitstellung einer Lösung eines Silbersalzes in wäßrigem Ammoniak,
(1) Zugabe von Phosphorsäure unter Ausfällung von Silberorthophosphat als Feststoff, der in Suspension enthalten wird, bis der pH-Wert der Suspension zwischen 6,0 und 8,0 liegt,
(m) Abtrennen des Feststoffes,
(n) Suspendieren des Feststoffes in deionisiertem Wasser,
(o) Zugabe von wässrigem Ammoniak zu der Suspension und Erwärmung der Mischung auf eine Temperatur im Bereich von 40-90°C, wobei die Menge an Ammoniak so bemessen ist, dass bei der gewählten Temperatur im Bereich von 40-90°C 1 Gew.% bis 80 Gew.% der Silberionen in Lösung gehen,
(p) Zugabe eines Reduktionsmittels bei der gewählten Temperatur im Bereich von 40-90°C in einer Menge, die ausreicht, die in Lösung befindlichen Silberionen zu metallischem Silber zu reduzieren, so dass ein teilreduziertes Silberorthophosphat als suspendierter Feststoff gebildet wird,
(q) Abtrennen des Feststoffes,
(r) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 20 µS/cm aufweist,
(s) Trocknen des Feststoffes,
(t) Einarbeiten des Feststoffes in eine Zusammensetzung zur topischen Anwendung.

3. Verfahren zur Herstellung einer antimikrobiell wirkenden kosmetischen und/oder pharmazeutischen Zusammensetzung zur topischen Anwendung, umfassend
(l) Bereitstellung einer Lösung eines Silbersalzes in wässrigem Ammoniak,
(m) Zugabe von Phosphorsäure unter Ausfällung von Silberorthophosphat als Feststoff, der in Suspension erhalten wird, bis der pH-Wert der Suspension zwischen 6,0 und 8,0 liegt,
(n) Abtrennen des Feststoffes,
(o) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 50 µS/cm aufweist,
(p) Suspendieren des Feststoffes in deionisiertem Wasser,
(q) Zugabe von wässrigem Ammoniak zur Suspension und Erwärmung der Mischung auf eine Temperatur im Bereich von 40-90°C, wobei die Menge an Ammoniak so bemessen ist, dass bei der gewählten Temperatur im Bereich von 40-90°C 1 Gew.% bis 80 Gew.% der Silberionen in Lösung gehen,
(r) Zugabe eines Reduktionsmittels bei der gewählten Temperatur im Bereich von 40-90°C in einer Menge, die ausreicht, die in Lösung befindlichen Silberionen zu metallischem Silber zu reduzieren, so dass ein teilreduziertes Silberorthophosphat als Feststoff gebildet wird,
(s) Abtrennen des Feststoffes,
(t) wiederholtes Waschen des Feststoffes mit Portionen von deionisiertem Wasser, bis die spezifische Leitfähigkeit bei 25°C der letzten Portion Waschwasser nach Abtrennung von dem Feststoff einen Wert unter 20 µS/cm aufweist,
(u) Trocknen des Feststoffes,
(v) Einarbeiten des Feststoffes in eine Zusammensetzung zur topischen Anwendung.

4. Verfahren nach Anspruch 2 oder 3, **dadurch gekennzeichnet, dass** das Reduktionsmittel aus Zuckern, Aldehyden, Hydrochinon, Weinsäure und Hydrazin ausgewählt ist.

5. Verfahren nach Anspruch 4, **dadurch gekennzeichnet, dass** das Reduktionsmittel Glucose ist.

6. Verfahren nach einem der Ansprüche 2 bis 5, **dadurch gekennzeichnet, dass** der Suspension des teilreduzierten Silberorthophosphats vor dem Abtrennen der Feststoffe Phosphorsäure zugesetzt wird, um einen pH-Wert im Bereich von 6,0 bis 8,0 einzustellen.

7. Verfahren nach einem der vorhergehenden Ansprüche, **dadurch gekennzeichnet, dass** ausschließlich Reinstwasser verwendet wird.

8. Verfahren nach einem der vorhergehenden Ansprüche, **dadurch gekennzeichnet, dass** der Feststoff durch Zentrifugieren abgetrennt wird.

9. Verfahren nach Anspruch 8, **dadurch gekennzeichnet, dass** der Feststoff zum Waschen in Reinstwasser dispergiert und anschließend durch Zentrifugieren abgetrennt wird.

10. Verfahren nach einem der vorhergehenden Ansprüche, **dadurch gekennzeichnet, dass** zunächst eine Grundzusammensetzung zur topischen Anwendung hergestellt und anschließend in diese der Feststoff eingemischt wird.

11. Verfahren nach einem der Ansprüche 1-9, **dadurch gekennzeichnet, dass** der Feststoff zunächst mit mindestens einem weiteren Bestandteil der Zusammensetzung vermischt wird, um eine Vormischung herzustellen, die anschließend durch Zugabe weiterer Bestandteile zu der fertigen Zusammensetzung zur topischen Anwendung weiter verarbeitet wird.

12. Verfahren nach einem der Ansprüche 1-11, **dadurch gekennzeichnet, dass** der getrocknete Feststoff in einer Menge von 0,001 Gew.% bis 10 Gew.%, bezogen auf das Gesamtgewicht der gebrauchsfertigen Zusammensetzung zur topischen Anwendung, in die Zusammensetzung eingearbeitet wird.

13. Verfahren nach Anspruch 12, **dadurch gekennzeichnet, dass** der Feststoff in einer Menge von 0,05 Gew.% bis 4 Gew.% in die Zusammensetzung eingearbeitet wird.

14. Antimikrobiell wirkende kosmetische und/oder pharmazeutische Zusammensetzung, **dadurch gekennzeichnet, dass** sie nach dem Verfahren gemäß einem der vorhergehenden Ansprüche erhältlich ist.

15. Zusammensetzung nach Anspruch 14, **dadurch gekennzeichnet, dass** sie eine Salbe ist.

16. Zusammensetzung nach Anspruch 14, **dadurch gekennzeichnet, dass** sie eine Creme ist.

17. Zusammensetzung nach Anspruch 14, **dadurch gekennzeichnet, dass** sie eine Lotion ist.

18. Zusammensetzung nach Anspruch 14, **dadurch gekennzeichnet, dass** sie ein Gel ist.

19. Zusammensetzung nach Anspruch 14, **dadurch gekennzeichnet, dass** sie eine Zahnpasta ist.

20. Zusammensetzung nach Anspruch 14, **dadurch gekennzeichnet, dass** sie ein Deodorant ist.

21. Verwendung der Zahnpasta gemäß Anspruch 19 zur Behandlung von Plaque und Paradontose.

22. Verwendung der Zusammensetzung gemäß einem der Ansprüche 14-18 zur Behandlung von Akne vulgaris.

23. Verwendung der Zusammensetzung gemäß einem der Ansprüche 14-18 zur Behandlung von Ekzemen.

24. Verwendung der Zusammensetzung gemäß einem der Ansprüche 14-18 zur Behandlung einer durch Herpesviren hervorgerufenen Erkrankung.

25. Verwendung der Zusammensetzung gemäß einem der Ansprüche 14-18 zur Behandlung einer Hautpilzerkrankung, insbesondere Fußpilz.

26. Verwendung der Zusammensetzung gemäß einem der Ansprüche 14-18 zur Behandlung von Wundinfektionen.

27. Verwendung der Zusammensetzung gemäß Anspruch 14 zur Schnupfenbehandlung.

28. Verwendung der Zusammensetzung gemäß einem der Ansprüche 14-18 zur Behandlung einer durch MRSA hervorgerufenen Infektion der Haut oder Schleimhaut.

29. Verwendung der Zusammensetzung gemäß Anspruch 14 zur Herstellung einer Zahnpasta zur Behandlung von Plaque und Paradontose.

30. Verwendung der Zusammensetzung gemäß einem der Ansprüche 14-18 zur Herstellung eines Arzneimittels zur Behandlung von Akne vulgaris.

31. Verwendung der Zusammensetzung gemäß einem der Ansprüche 14-18 zur Herstellung eines Arzneimittels zur Behandlung von Exzemen.

32. Verwendung der Zusammensetzung gemäß einem der Ansprüche 14-18 zur Herstellung eines Arzneimittels zur Behandlung einer durch Herpesviren hervorgerufenen Erkrankung.

33. Verwendung der Zusammensetzung gemäß einem der Ansprüche 14-18 zur Herstellung eines Arzneimittels zur Behandlung einer Hautpilzerkrankung, insbesondere Fußpilz.

34. Verwendung der Zusammensetzung gemäß einem der Ansprüche 14-18 zur Herstellung eines Arzneimittels zur Behandlung von Wundinfektionen.

35. Verwendung der Zusammensetzung gemäß Anspruch 14 zur Herstellung eines Arzneimittels zur Schnupfenbehandlung.

36. Verwendung der Zusammensetzung gemäß einem der Ansprüche 14-18 zur Herstellung eines Arzneimittels zur Behandlung einer durch MRSA hervorgerufenen Infektion der Haut oder Schleimhaut.

37. Verfahren nach einem der Ansprüche 1-13, **dadurch gekennzeichnet, dass** der Feststoff nicht auf ein anorganisches Trägermaterial aufgebracht wird.

38. Verfahren nach Anspruch 37, **dadurch gekennzeichnet, dass** der Feststoff nicht auf ein Oxid des Titans, Magnesiums, Aluminiums, Siliziums, Cers, Zirconiums, Hafniums, Niobs oder Tantals oder auf Calciumhydroxylapatit oder Bariumsulfat oder auf eine Mischung derselben aufgebracht wird.

39. Verfahren nach Anspruch 38, **dadurch gekennzeichnet, dass** der Feststoff nicht auf Titandioxid aufgebracht wird.

40. Zusammensetzung nach einem der Ansprüche 14-20, **dadurch gekennzeichnet, dass** sie frei von einem anorganischen Trägermaterial für den Feststoff ist.

41. Zusammensetzung nach einem der Ansprüche 14-20, **dadurch gekennzeichnet, dass** sie frei von Oxiden des Titans, Magnesiums, Aluminiums, Siliciums, Cers, Zirconiums, Hafniums, Niobs und Tantals und frei von Calciumhydroxylapatit und Bariumsulfat ist.

42. Zusammensetzung nach Anspruch 41, **dadurch gekennzeichnet, dass** sie frei von Titandioxid ist.
